# EUROPEAN PATENT SPECIFICATION

(11) **EP 2 029 353 B1**
(45) Date of publication and mention of the grant of the patent: **16.03.2016**
(21) Application number: 07732918.3
(22) Date of filing: 22.05.2007
(51) Int. Cl.: B32B 5/18, A01N 25/34, E05B 1/00, A61L 2/16

(54) **SURFACE MOUNTABLE DELIVERY DEVICE**
OBERFLÄCHENMONTIERBARE ABGABEVORRICHTUNG
DISPOSITIF DE LIVRAISON À MONTER EN SURFACE

(30) Priority: 22.05.2006 GB 0610096
(43) Date of publication of application: 04.03.2009
(73) Proprietor: SURFACESKINS LIMITED, West Yorkshire LS2 9JT (GB); Zelo Creative Limited, Leeds, West Yorkshire LS1 4HT (GB)
(72) Inventor: RUSSELL, Stephen, John, HG2 8QE (GB); TIPPER, Matthew, James, YO26 8RA (GB); RATHOD, Manoj Kantilal, Chhaganlal, LS27 0LB (GB); WALKER, Adam, David, West Yorkshire LS23 7RA (GB); SCOTT-HARDEN, Simon, Geoffrey, John, Cumbria, CA11 0UU (GB)
(74) Representative: White, Nicholas John
(86) International application number: PCT/GB2007/001897
(87) International publication number: WO 2007/135424

(56) References cited:
- DE-A1- 3 026 258
- DE-A1- 10 158 286
- DE-U1- 20 310 052
- US-A- 4 832 942
- US-A1- 2002 041 824
- US-A1- 2003 203 015

## Description

### FIELD OF THE INVENTION

The present invention relates to a novel means of delivering material, such as liquids or gels over a sustained period of time.

In particular the invention provides a novel surface mountable delivery device capable of protecting surfaces from infection and/or disinfecting surfaces, e.g. door handles, etc. and/or delivering, for example, an antimicrobial agent as an aid in preventing and/or hindering the spread of infection.

### BACKGROUND

It is widely recognised that there is a major problem with the spread of germs from one place to the next, as people/animals and organisms touch one surface and another. Contact - hence the word "contagious disease", is the most common way that infection can spread from one individual to another. This transmission of microorganisms, e.g. germs, bacteria, etc. provides a health hazard as microorganisms present on the hands/skin/body of people/organisms, are transferred onto the surfaces of items such as door handles/door knobs/door push plates/ door rails/door leavers/counters/work surfaces/sinksltaps/car handles/towel/rails/bath rails/sink rails/stair lift rails/banisters, to name but a very small selection of surfaces that are frequently touched/contacted by a large number of individuals, in a short space of time. Such surfaces are then touched by the hands/skin/body of other person/organisms and these microorganisms may be picked up by this new individual. If this new individual then makes contact with an open skin surface, such as a cut/abrasion or makes oral contact, there is a strong chance that the bacteria could be transmitted into their body system. Once in the body system, these bacteria could cause a: cold, flu, mumps/missals, conjunctivitis, diarrhoea, bronchitis, dermatological disorders or sickness for example.

However, these are not the worst-case results of transmitting bacteria from one individual to the next, through surface contact. There are many nasty diseases that can be considered contagious; one of the most deadly viruses that can be transmitted by surface contact is methicillin-resistant *Staphylococcus aureus* (MRSA).

The organism *Staphylococcus aureus* is found on many individuals skin and seems to cause no major problems. However, if *Staphylococcus aureus* gets inside the body, for instance under the skin or into the lungs, it can cause important infections, such as pneumonia. Individuals who carry this organism may be or appear healthy, have no problems whatever and are considered simply to be carriers of the organism.

Methicillin-resistant *Staphylococcus aureus* (MRSA) is a specific strain of the *Staphylococcus aureus* bacterium that has developed antibiotic resistance to all penicillins, including methicillin. MRSA. Methicillin was an antibiotic used many years ago to treat patients with Staphylococcus aureus infections. It is now no longer used except as a means of identifying this particular type of antibiotic resistance. Individuals can become carriers of MRSA in the same way that they can become a carrier of ordinary *Staphylococcus aureus* which is by physical contact with the organism, for example, if the organism is on the skin then it can be passed around by physical contact. If the organism is in the nose or is associated with the lungs rather than the skin then it may be passed around by droplet spread from the mouth and/or nose.

MRSA organisms are often associated with patients in hospitals but can also be found on patients not in a hospital. Usually it is not necessary to do anything about MRSA organisms. However if MRSA organisms are passed on to someone who is already ill, then a more serious infection may occur in that individual. When patients with MRSA are discovered in a hospital, the hospital will usually try to prevent it from passing around to other patients. This is known as infection control. Measures to prevent the spread of organisms from one person to another are called isolation or infection control. The type of infection control or isolation required for any patient depends, *inter alia*, on the organism, where the organisms are found on/in an individual and the patient. The most important type of isolation required for MRSA is what is called "contact isolation". This type of isolation requires everyone in contact with the patient to be very careful about hand washing after touching either the patient or anything in contact with the patient.

One very important point about infections and viruses that spread in hospitals, is that about 10% of infections in the UK's public hospitals have been estimated to be airborne. This means that approximately 90% of infections are therefore transmitted in other ways, such a through contact with surfaces and other individuals.

Biosecurity is a term that covers the actions and measures needed to be taken to safeguard individuals from diseases caused by viral, bacterial and fungal infections. Biosecurity is essential against the fight of these contagious diseases.

Thus, it is highly desirable to provide a means of reducing the spread of microorganism, e.g. in, hospitals, (where surfaces are regularly touched by many people), doctors waiting rooms and doors, public houses including door handles and furniture, veterinary buildings and doors etc, as well as office equipment/computer keyboards/mice etc. This is just a minute example of the applications for this invention, but it does however represent some of the most beneficial areas to aim the invention, to gain the best results against the spread of contagious diseases. A number of attempts have been made to address this.

Thus, for example, US Patent No. 5,407,685 describes a film for releasing an antimicrobial agent, oxygen and a medicament comprising a flexible, porous layer and dry reagents which react in the presence of a diluent.

US Patent No. 5,882,667 describes a sterile rigid sleeve produced from a mixture of a plastic and an antimicrobial agent form of an elongated rigid cylinder which is the shaft of a writing pen.

U.S. Patent No. 6,298,521 discloses a housing containing a sublimable disinfectant, the housing having an adhesive on the rear end for securing it to the backing plate behind a door knob. The housing has circumferential slots in the front end so that the sublimable disinfectant vapours may reach the door knob.

US patent No. 6,863,960 describes a user-activatible substance delivery system for, e.g. soaps, fragrances, lotions, adhesives, and the like comprising a first web and a second web joined together in a face to face relationship and being joined about their respective peripheries to define a void space therein; which houses a fluid. US Patent No. 6,821,325 describes a multi-surface antibacterial protective device comprising, an under layer of material and an over a layer of a permeable material and a cavity disposed between the two. The cavity houses an antibacterial solution. Whilst US '325 addresses the same problem as the present invention, there is little or no control over the seepage of the antibacterial agent from the cavity. Furthermore, when a liquid is used in the cavity, settlement of the liquid to the bottom of the system occurs, particularly when it is oriented vertically in use. Failure to address settlement causes a variation in the amount of liquid delivered from top to bottom, which becomes more pronounced as the system is progressively emptied of solution. Clearly, such a variation in the delivery rate across the system is a major concern where the system is applied to walls and door plates, etc. and this has been addressed in the new design.

Therefore, there remains a long felt want for a suitable material which is capable of acting as a barrier material and a controllable reservoir for one or more active ingredients, bactericide, viruscide, fungicide, etc.

### STATEMENT OF INVENTION

Therefore, according to a first aspect of the invention we provide a surface mountable delivery device which has a multi layer construction comprising an elastic liquid permeable support layer adjacent to a porous reservoir layer, the porous reservoir layer being provided with a backing layer and wherein the liquid permeable layer is only rendered permeable to liquid on compression of the device.

The liquid permeable support layer is, in use, a top layer or an outer layer. The layer is a liquid permeable film or membrane. The film may be a porous film or a perforated film, e.g. a micro perforated film. It is preferentially, a microperforated film in which the Permeability is dependent upon the transverse pressure that is applied to the entire construction in use. A variety of films may be used, thus, the film may be selected from any conventionally known film-forming polymer. Preferably, the film will be compatible with printing. Also, the film will be nondegradable and/or soluble in water or when in contact with an alcohol or an oil-water emulsion. It is especially importantly that the printed surface should be insensitive to alcohol, e.g. ethanol, which forms the basis of many commercially available antibacterial formulations e.g. Cutan^{®}.

Thus, the perforated support film may be hydrophobic polymer, such as polyethylene (PE), polypropylene (PP) or polyethylene terephthalate (PET) and copolymers thereof.

The pore openings of the microperforated film may vary depending, *inter alia,* upon the nature and composition of the antibacterial formulation present in the porous reservoir layer. Thus the pores of the microperforated film may consist of sub-micron dimensions, however, preferentially, a microperforated film with openings of from 20-500µm diameter may be used, that is for example more than 50% of the pores have a diameter in the range of from 20-500µm, preferably more than 70%, more preferably more than 90%. The support layer, e.g. the perforated film has some elastic properties so that the pore openings may open to their maximum extent due to the fluid forces introduced by evacuation of the liquid/gel from the porous reservoir layer and then self-close to some extent by elastic recovery of the film when the compression is removed from the system and the fluid flow ceases. Nevertheless, it should be understood that perforated high modulus films may also be suitable. Furthermore, it may be advantageous for each of the layers in the multi layer device to possess elastic properties.

The porous reservoir layer may comprise a variety of materials, such as one or more of a foam, a woven material or nonwoven material, although a nonwoven material may be preferred. The porous reservoir may comprise a composite material and/or may comprise a multilayer material. Such a nonwoven material may be composed of a variety of materials, such as, cellulose pulp or other absorbent fibrous material, capable of holding liquid within and between the pores of adjacent fibres. The porous reservoir preferentially has a high capillary action as this is especially advantageous when the device is in use in a vertical position. In a further alterative the porous layer may comprise a plurality of chambers each containing a porous material as hereinbefore described. The porous reservoir layer may preferably be contiguous with the permeable support layer.

A backing third layer may be provided on the porous reservoir layer that prevents penetration of active liquid through and which prevents evaporation. This backing layer is preferably an unperforated and non-permeable film composed of a hydrophobic polymer. Exemplary hydrophobic polymers include polyethylene (PE), polypropylene (PP) or polyethylene terephthalate (PET) and copolymers thereof.

The device of the invention may include means for attaching the device to a surface.

This may comprise an attaching means, e.g. a Velcro^{®} arrangement or may comprise one or more adhesive surfaces. The delivery device may be design to attaché to the surface or to attach to itself, by wrapping around a surface. In a preferred embodiment the attaching means comprises an adhesive, for example, the backing layer is coated or substantially coated with an adhesive layer, e.g. a pressure-sensitive adhesive, to enable fixation of the surface mountable delivery device to various surfaces. Such an adhesive layer may be applied to the backing layer or alternatively the backing layer may itself comprise an adhesive provided that such an adhesive is contiguous over the surface of the porous layer and prevents penetration of active liquid through and which prevents evaporation. Alternatively, if the porous layer is not contiguous with the permeable layer, adhesive may be applied to the surface of the permeable layer that is exposed adjacent to the porous layer. The adhesive is preferably a pressure sensitive adhesive which may optionally be alcohol soluble, thus enabling it to be removed from surfaces when the delivery device of the invention is removed. The pressure sensitive adhesive may be an acrylic adhesive such as an acrylate ester copolymer adhesive formed by the copolymerization of 2-ethyl-hexyl acrylate, butyl acrylate and acrylic acid. Alternatively, the adhesive layer may be an adhesive such as polyvinyl alkyl ether adhesive.

In a preferred aspect of the invention the liquid permeable first layer and the backing layer may be capable of thermoplastic bonding, e.g. ultrasonic joining.

In use, the porous reservoir layer contains an active agent, e.g. is impregnated or impregnatable with any conventionally known active agents, such as, an active agent selected from one or more of an antimicrobial agent, a medicament, a cosmetic a perfume, and a deodorant. In a preferred aspect of the present invention the active agent is an antimicrobial agent. The active agent may be present in a form selected from form selected from solid, liquid, gel, suspension, emulsion and microencapsulated. Preferably, the active agent will be present in liquid or gel form. The term antimicrobial will be well understood by the person skilled in the art and shall include antibacterial, antifungal and antiviral compositions; and mixtures thereof. The term antibacterial shall include bactericidal and bacteristatic compositions.

Any conventionally known antibacterial compositions may be used, including, for example, alcohols, such as "surgical alcohols", e.g. ethanol, 1-propanol and 2-propanol/isopropanol; or mixtures thereof. Other antibacterial compositions which may be mentioned include, quaternary ammonium compounds, such as benzalkonium chloride, chlorhexidine, iodine, phenol (carbolic acid) compounds or silver compounds; or mixtures thereof. A preferred antibacterial composition is an alcohol, such as that commercially available as Cutan^{®} from Deb Limited in the UK. An especially preferred antibacterial agent has an alcohol content of from 58 to 78% w/w, preferably from 68 to 72% w/w and most preferably alcohol content of 70% w/w.

It is an especially preferred feature of the present invention that the device is adapted to remain bacteristatic during its lifetime.

A further preferred antibacterial agent is one which is capable as acting as bactericidal agent or bacteristatic agent, e.g. to MRSA. The antibacterial agent is especially a bactericidal agent or a bacteristatic agent to one or more of MRSA, MSSA, Necrotizing fasciitis, Escherichia coli, NorA, Clostridium difficile, Norovirus, enterococcus faecium and pseuomonas aruginosa. For example, vancomycin, methicillin, etc.

### Examples of antifungal agents include, boric acid, or combined antibacterial and antifungal agents, such as triclosan,

The total loading of antimicrobial agent in the porous reservoir layer is dependent upon, *inter alia,* the fabric thickness and density. This determines the total pore volume or porosity of the layer and therefore its absorbent capacity. The delivery rate may be controlled by the compression resistance of the fabric, the total loading of the active agent, e.g. liquid, the perforated hole sizes in the support layer and the viscosity of the liquid. The latter can be controlled by additives, such as a thickener, if required. However, impregnation of the porous reservoir layer is typically by saturation, thus allowing the highest loading possible and providing the longest duration ofactivity, e.g. antimicrobial activity.

Thus, according to a further aspect of the invention we provide a surface mountable delivery device as hereinbefore described wherein the porous reservoir layer is loaded with an active agent. The active agent is preferentially an antimicrobial agent as hereinbefore described.

In the material of the invention the permeable support layer may also be provided with a removable cover layer as projection. Such a cover layer may be capable of preventing the pores from becoming blocked prior to putting the surface skin in place where the antimicrobial activity is required. Similarly, if the backing layer is provided with an adhesive layer then the adhesive layer and/or the backing layer may also be provided with a cover layer. The removable cover may be, for example, a silicone coated release paper. Alternatively, the whole delivery device may be presented in a sealed package.

The surface mountable delivery device of the invention may be made up in a variety of forms. By way of illustration, such forms include, but shall not be limited to, stickers, tapes, pads, tubes, socks and the like.

The surface mountable delivery device of the invention may include an indicator, such as an obsolescence indicator. This may be a time activated indicator or it may be adapted to provide an indication of the internal state of the reservoir layer.

The device may also be substantially biodegradable and/or compostable.

In a further preferred embodiment of the invention we provide a method of preventing the transmission of microrganisms which comprise the use or application of a surface mountable delivery device as hereinbefore described.

The surface mountable delivery device of the present invention is advantageous over prior art devices since the present invention does not use a filled internal cavity but rather a porous material e.g. a nonwoven material, with a high internal surface area (due to the fibres) and a porosity of ca. 75-99% to store the liquid, gel, etc. The use of, e.g. a nonwoven material, prevents the settlement of the liquid to the bottom of the system. The liquid is held between the fibre surfaces and spreads through the middle layer partly by means of capillary action, g a "wicking" effect. The liquid is therefore physically held between fibres and does not flow out through the support layer film unless the porous layer is compressed.

Furthermore, in the design of the present invention the solution does not seep through the permeable layer but is transported only by forced flow (induced by applied pressure) through the film. Compression of the porous material within the system effectively squeezes out the solution through the outer film support layer. The film support layer is permeable to liquid only when compressed. This helps to prevent evaporation and therefore renders the system active for a period of 1 day to a maximum of about 14 days. The support layer film surface of the system is therefore intended to be completely dry unless the system is compressed to deliver the solution to the contact point.

The invention will now be referred to by the following figures 1-10, which show/ represent various forms/states and designs, the invention could take and in which
Figure 1 is a cross-section of a surface skin of the invention;
Figure 2 is a cross-section of a surface skin of the invention provided with an adhesive layer;
Figure 3 is a cross-section of a surface mountable delivery device of the invention in which the permeable layer and the backing layer are sealed together;
Figure 4 is a cross-section of a surface mountable delivery device of the invention with adhesive and a removable cover layer;
Figure 5 is a schematic representation of a cruciform pad applied to a door handle;
Figure 6 is a schematic representation of tape applied to a door handle;
Figure 7 is a schematic representation of a tube or sock applied to a door handle;
Figure 8 is a schematic representation of a round shape applied to a door knob;
Figure 9 is a schematic representation of a tape applied to a door knob;
Figure 10 is a schematic representation of a pad applied to a push plate;
Figure 11 is a schematic representation of a pad with the cover being removed;
Figure 12 is a schematic representation of a pad applied to a push plate; and
Figure 13 is a schematic representation of a multilayered pad applied to a door handle.

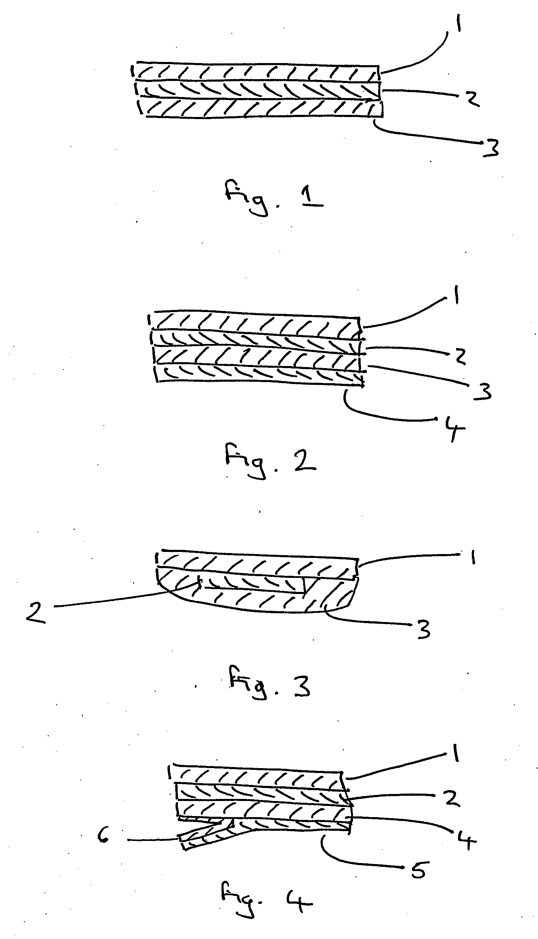

**Figure 1****:** A surface skin material comprises a liquid permeable support layer (1) adjacent to a porous reservoir layer (2), the porous reservoir layer (2) being provided with a backing layer (3).

**Figure 2****:** A surface skin material comprises a liquid permeable support layer (1) adjacent to a porous reservoir layer (2), the porous reservoir layer (2) being provided with a backing layer (3) and the backing layer (3) being provided with an adhesive layer (4).

**Figure 3****:** A surface skin material comprises a liquid permeable support layer (1) adjacent to a non-contiguous porous reservoir layer (2), the porous reservoir layer (2) being provided with a backing layer (3) which also may be sealed against the support layer (1).

**Figure 4****:** A surface skin material comprises a liquid permeable support layer (1) adjacent to a porous reservoir layer (2), the porous reservoir layer (2) being provided with an adhesive layer (4) and the adhesive layer (4) being provided with a releasable cover or protector layer (5 & 6).

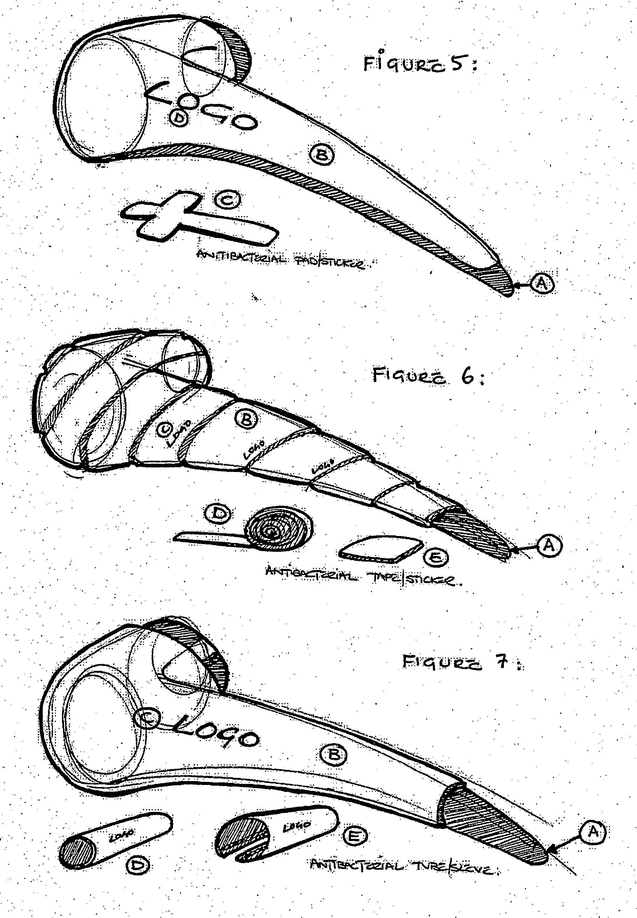

**Figure 5****:** Represents an antibacterial/viral pad/patch/sticker/plaster/sock, which may be cross shaped/triangular shape/rectangular and/or of a tape like construction, which may or may not have a form of adhesive/sticky pad or patch on the back. This antibacterial pad/patch/sticker/plaster may or may not be stretchy/elastic in nature so that it may/may not contort and distort to best form around a desired surface, such as a door handle for example, as shown by (B). (See list at the end of the figures for other desired surfaces). (A) Shows the invention in the context of a door handle, however, this could be any form of surface/product. (C) Shows one example of the shape of the invention, although it could be square, circular, rectangular, triangular, oblong, tubular, elliptical, or any other shape/construction. (D) Denotes that the invention may or may not have a logo anywhere on the invention, which may be a health care organisation/company or any other name/brand.

This antibacterial/anti viral pad/patch/sticker/plaster may be made from a polymer, composite polymer, rubber, plastic, woven fabric and or injection moulded, extruded, woven, pressed, die cut, stamped, blow moulded method.

**Figure 6****:** Represents the invention in a different form. (B) Shows how the antibacterial/anti viral pad/patch/sticker/plaster/sock may or may not be in the form of tape/webbing/strapping/strip which may or may not have a form of adhesive/sticky pad or patch on the back. (A) Shows the invention in the context of a door handle, however, this could be any form of surface/product as listed below. (C) Shows that it may or may not have a logo. (D) Shows that it may/may not be in the form of a tape/webbing/strapping/strip. (E) Shows that it may/may not be in the shape of a viral pad/patch/sticker/plaster.

This antibacterial/anti viral pad/patch/sticker/plaster/sock may be made from a polymer, composite polymer, rubber, plastic, and or injection moulded, extruded, woven, pressed, die cut, stamped, blow moulded method.

**Figure 7****:** Shows the invention in a different form. (B) Shows the antibacterial/anti viral pad/patch/sticker/plaster in the form of a tube/sock which may or may not be open or closed as a tube. This form of the invention may be sleeved over the desired surface, which could be a door handle for one example, shown by (A). (C) Denotes that the invention may or may not have a logo. (D) Shows the invention in an open sock state, which is essentially a tube of material, which may or may not be elasticated/expandable, so that when it sleeves over a surface it can contract to secure/lock in place. It may or may not also have adhesive/sticky surfaces on it to help secure in a position. (E) Shows the invention in an open state. In this state the invention can be opened up to wrap around a surface and then closed back together with a form of attachment on the invention. This form of attachment may or may not be an adhesive/sticky surface, clips, Velcro, etc.

This antibacterial/anti-viral pad/patch/sticker/plaster/sock may be made from a polymer, composite polymer, rubber, plastic, and or injection moulded, extruded, woven, pressed, die cut, stamped, blow moulded method.

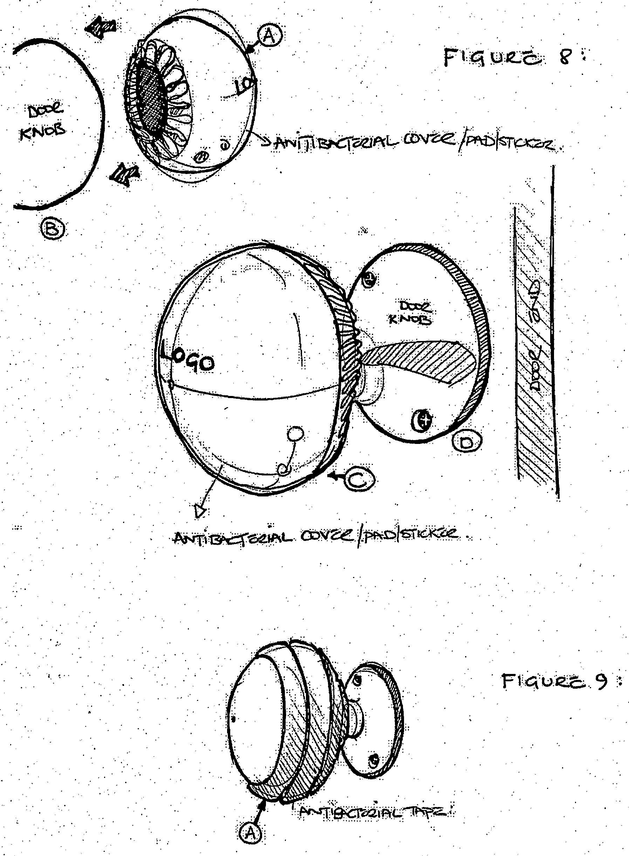

**Figure 8****:** Shows the invention in another form and in the context of a door knob/cylindrical handle/rounded surface. (A) Represents the antibacterial/anti viral pad/patch/sticker/plaster/sock and (B) represents a round surface such as a doorknob. (C) Shows the invention in contact with (D) the door knob/cylindrical handle/rounded surface, which will have a form of keeping it attached to the desired surface. This may through a sticky/adhesive surface or an elasticised/expandable material which contracts to grip the desired surface.

This antibacterial/anti viral pad/patch/sticker/plaster/sock may be made from a polymer, composite polymer, rubber, plastic, and or injection moulded, extruded, woven, pressed, die cut, stamped, blow moulded method.

**Figure 9****:** Shows how the antimicrobial pad, patch, sticker, plaster, sock attaches to a door knob/cylindrical handle/rounded surface/ curved surface if made in a pad, patch, sticker, plaster, sock, tape, webbing, strapping, format.

This antibacterial/anti viral pad/patch/sticker/plaster/sock may be made from a polymer, composite polymer, rubber, plastic, and or injection moulded, extruded, woven, pressed, die cut, stamped, blow moulded method.

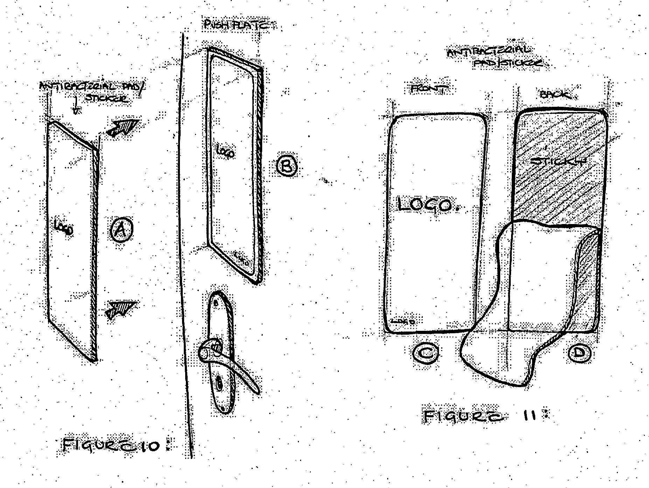

**Figure 10****:** Shows an antibacterial/anti viral pad/patch/sticker/plaster/sock, which may or may not fit over/onto a push plate on a door for example. (A) Shows the invention, which may then fit over/onto (B), which represents a push surface. This push surface may be on any product/surface. For this example it is shown in the context of a door. It may however be attached to any surface.

This antibacterial/anti viral pad/patch/sticker/plaster may be made from a polymer, composite polymer, rubber, plastic, and or injection moulded, extruded, woven, pressed, die cut, stamped, blow moulded method.

**Figure 11****:** Shows an antibacterial/anti viral pad/patch/sticker/plaster/sock which may or may not fit over/onto a push plate on a door for example. (C) Shows the front of the invention, which may or may not include a logo. (D) Shows the back of the invention, which has a form of attaching it to a surface. This may or may not be in the form of an adhesive/sticky surface.

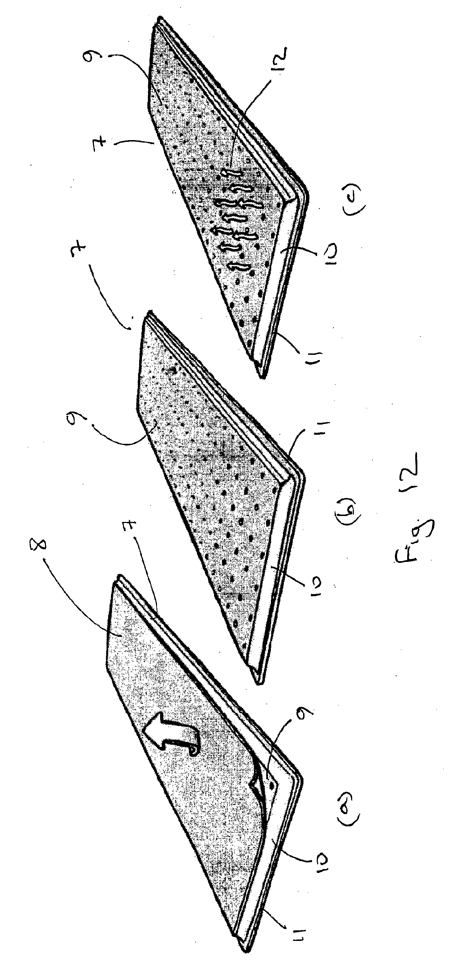

**Figure 12:** Figure 12(a) shows an antibacterial pad (7) with an "activation" film cover layer (8) in place. In figure 12(b) the "activation" film cover layer (8) is removed, exposing the perforated support layer (9). The porous reservoir (10) and backing layer (11) are also shown. Figure 12(c) schematically represents liquid (12) passing through the perforated support layer (9).

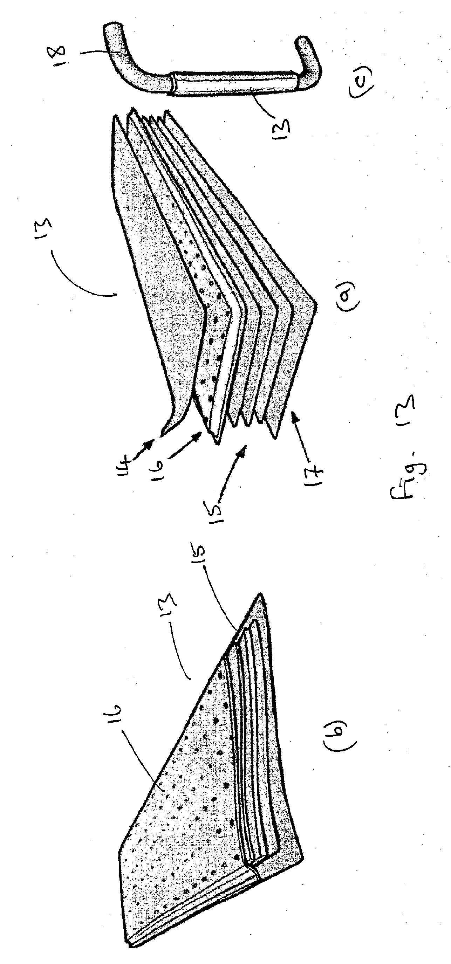

**Figure 13:** Figure 13(a) shows an antibacterial pad (13) with an "activation" film cover layer (14) in place and a multilayered reservoir (15). In figure 13(b) the "activation" film cover layer (14) is removed, exposing the perforated support layer (16). The backing layer (17) is also shown. Figure 13(c) shows the pad (13) fixed to a door handle (18).

This antibacterial/anti viral pad/patch/sticker/plaster may be made from a polymer, composite polymer, rubber, plastic, and or injection moulded, extruded, woven, pressed, die cut, stamped, blow moulded method.

All the examples of the various forms of the invention may or may not have an anti viral killing substance in/on/or impregnated within and are designed to be attached to any surface.

In all of the designs shown from figures 5 to 11 the pad, patch, sticker, plaster, tube, sock, could be applied to the surface of: door handles/door knobs/push plates/push pads/grab handles/grab rails/banister rails/towel rails/ work surfaces/taps/knobs/dials/buttons/steering wheels/chairs/chair arms/ doors/computer equipment/computer mice/keyboards/electronic equipment/public transport/public seats/bus stops/street furniture/glass surfaces/wood surfaces/metal surfaces/plastic surfaces/office equipment/pubic house furniture and fittings/toilet handles/light fittings/plugs/plug sockets/public furniture/public transport/hospital equipment/surgical instruments/doctor waiting rooms/surgery clinic surfaces/veterinary clinic surfaces and equipment/tools and any surface touched by organisms/individuals/people/animals.

## Claims

1. A surface mountable delivery device which has a multi layer construction comprising an elastic liquid permeable support layer adjacent to a porous reservoir layer, the porous reservoir layer being provided with a backing layer and wherein the liquid permeable layer is only rendered permeable to liquid on compression of the device.

2. A surface mountable delivery device according to claim 1 wherein the liquid permeable support layer is a perforated film.

3. A surface mountable delivery device according to claim 1 wherein the liquid permeable support layer is a porous film.

4. A surface mountable delivery device according to claim 1 wherein the elastic liquid permeable support layer has pores that may open to their maximum extent due to the fluid forces introduced by evacuation of liquid or gel from the porous reservoir layer on compression of the device and then self-close to some extent by elastic recovery of the layer when the compression is removed and fluid flow ceases.

5. A surface mountable delivery device according to claim 1 wherein the liquid permeable support layer comprises a film-forming polymer.

6. A surface mountable delivery device according to claim 1 wherein the permeable support layer is non-degradable and/or soluble in water or when in contact with an alcohol or an oil-water emulsion.

7. A surface mountable delivery device according to claim 3 wherein the pore openings in the liquid permeable support layer consist of openings of from 20-500 µm diameter.

8. A surface mountable delivery device according to claim 1 wherein the liquid permeable support layer comprises a hydrophobic material.

9. A surface mountable delivery device according to claim 1 wherein the porous reservoir layer comprises one or more of a foam, a woven material or a nonwoven material.

10. A surface mountable delivery device according to claim 1 wherein the backing layer comprises a hydrophobic polymer.

11. A surface mountable delivery device according to claim 1 wherein the delivery device includes means for attaching the system to a surface.

12. A surface mountable delivery device according to claim 13 wherein the means for attaching the system to a surface comprises an adhesive coating.

13. A surface mountable delivery device according to claim 1 wherein the porous reservoir layer contains an active agent.

14. A surface mountable delivery device according to claim 1 wherein the support layer contains an antimicrobial agent.

15. A surface mountable delivery device according to claim 1 wherein the reservoir layer comprises a liquid composition that is unable to flow from the porous reservoir material until the reservoir is compressed.

16. A method of preventing the transmission of micro-organisms which comprises the use or application of a surface mountable delivery device according to any one of claims 1 to 15.

## Patentansprüche

1. Abgabevorrichtung, die an einer Oberfläche befestigbar ist, die eine mehrschichtige Konstruktion aufweist, die eine elastische flüssigkeitspermeable Trägerschicht anliegend an eine poröse Speicherschicht umfasst, wobei die poröse Speicherschicht mit einer Unterstützungsschicht zur Verfügung gestellt ist und wobei die flüssigkeitspermeable Schicht nur auf die Kompression der Vorrichtung für die Flüssigkeit permeabel wird.

2. Abgabevorrichtung, die an einer Oberfläche befestigbar ist, nach Anspruch 1, wobei die flüssigkeitspermeable Trägerschicht eine perforierte Folie ist.

3. Abgabevorrichtung, die an einer Oberfläche befestigbar ist, nach Anspruch 1, wobei die flüssigkeitspermeable Trägerschicht eine poröse Folie ist.

4. Abgabevorrichtung, die an einer Oberfläche befestigbar ist, nach Anspruch 1, wobei die elastische flüssigkeitspermeable Trägerschicht Poren aufweist, die sich aufgrund der Kräfte der Flüssigkeit, die durch Evakuation von Flüssigkeit oder Gel von der porösen Speicherschicht auf die Kompression der Vorrichtung maximal öffnen und sich dann zu einem gewissen Grad durch Elastizitätsrückgewinnung der Schicht wieder selbst schließen, wenn die Kompression beendet wird und der Flüssigkeitsstrom aufhört.

5. Abgabevorrichtung, die an einer Oberfläche befestigbar ist, nach Anspruch 1, wobei die flüssigkeitspermeable Trägerschicht ein filmbildendes Polymer umfasst.

6. Abgabevorrichtung, die an einer Oberfläche befestigbar ist, nach Anspruch 1, wobei die permeable Trägerschicht in Wasser oder beim Kontakt mit Alkohol oder einer Öl-Wasser-Emulsion nicht abbaubar und/oder löslich ist.

7. Abgabevorrichtung, die an einer Oberfläche befestigbar ist, nach Anspruch 3, wobei die Porenöffnungen in der flüssigkeitspermeablen Trägerschicht aus Öffnungen mit einem Durchmesser von 20 bis 500 µm bestehen.

8. Abgabevorrichtung, die an einer Oberfläche befestigbar ist, nach Anspruch 1, die flüssigkeitspermeable Trägerschicht ein hydrophobes Material umfasst.

9. Abgabevorrichtung, die an einer Oberfläche befestigbar ist, nach Anspruch 1, wobei die poröse Speicherschicht eines oder mehrere umfasst aus einem Schaum, einem gewebten Material oder einem Vliesmaterial.

10. Abgabevorrichtung, die an einer Oberfläche befestigbar ist, nach Anspruch 1, wobei die Unterstützungsschicht ein hydrophobes Polymer umfasst.

11. Abgabevorrichtung, die an einer Oberfläche befestigbar ist, nach Anspruch 1, wobei die Abgabevorrichtung Mittel zum Befestigen des Systems an einer Oberfläche beinhaltet.

12. Abgabevorrichtung, die an einer Oberfläche befestigbar ist, nach Anspruch 13, wobei das Mittel zum Befestigen des Systems an einer Oberfläche eine Klebebeschichtung umfasst.

13. Abgabevorrichtung, die an einer Oberfläche befestigbar ist, nach Anspruch 1, wobei die poröse Speicherschicht ein aktives Mittel enthält.

14. Abgabevorrichtung, die an einer Oberfläche befestigbar ist, nach Anspruch 1, wobei die Trägerschicht ein antimikrobielles Mittel enthält.

15. Abgabevorrichtung, die an einer Oberfläche befestigbar ist, nach Anspruch 1, wobei die Speicherschicht eine flüssige Zusammensetzung umfasst, die nicht aus dem porösen Speichermaterial fließen kann, solange der Speicher nicht komprimiert wird.

16. Verfahren zur Verhinderung der Übertragung von Mikroorganismen, das die Verwendung oder Anwendung einer Abgabevorrichtung, die an einer Oberfläche befestigbar ist, nach einem der Ansprüche 1 bis 15 umfasst.

## Revendications

1. Dispositif de distribution pouvant être monté en surface qui a une structure multicouche comprenant une couche de support élastique perméable au liquide adjacente à une couche de réservoir poreuse, la couche de réservoir poreuse étant munie d'une couche dorsale et dans lequel la couche perméable au liquide est seulement rendue perméable au liquide lors de la compression du dispositif.

2. Dispositif de distribution pouvant être monté en surface selon la revendication 1, dans lequel la couche de support perméable au liquide est un film perforé.

3. Dispositif de distribution pouvant être monté en surface selon la revendication 1, dans lequel la couche de support perméable au liquide est un film poreux.

4. Dispositif de distribution pouvant être monté en surface selon la revendication 1, dans lequel la couche de support élastique perméable au liquide a des pores qui peuvent s'ouvrir à leur étendue maximale en raison des forces fluidiques introduites par l'évacuation de liquide ou de gel depuis la couche de réservoir poreuse lors de la compression du dispositif et ensuite se fermer jusqu'à une certaine mesure par le rétablissement élastique de la couche quand la compression est enlevée et que le flux de fluide cesse.

5. Dispositif de distribution pouvant être monté en surface selon la revendication 1, dans lequel la couche de support perméable au liquide comprend un polymère filmogène.

6. Dispositif de distribution pouvant être monté en surface selon la revendication 1, dans lequel la couche de support perméable n'est pas dégradable et/ou soluble dans l'eau ou quand elle est en contact avec un alcool ou une émulsion huile-eau.

7. Dispositif de distribution pouvant être monté en surface selon la revendication 3, dans lequel les ouvertures de pore dans la couche de support perméable au liquide consistent en ouvertures de 20 à 500 µm de diamètre.

8. Dispositif de distribution pouvant être monté en surface selon la revendication 1, dans lequel la couche de support perméable au liquide comprend une matière hydrophobe.

9. Dispositif de distribution pouvant être monté en surface selon la revendication 1, dans lequel la couche de réservoir poreuse comprend une ou plusieurs d'une mousse, d'une matière tissée ou d'une matière non tissée.

10. Dispositif de distribution pouvant être monté en surface selon la revendication 1, dans lequel la couche dorsale comprend un polymère hydrophobe.

11. Dispositif de distribution pouvant être monté en surface selon la revendication 1, dans lequel le dispositif de distribution inclut un moyen pour attacher le système à une surface.

12. Dispositif de distribution pouvant être monté en surface selon la revendication 13, dans lequel le moyen pour attacher le système à une surface comprend un revêtement adhésif.

13. Dispositif de distribution pouvant être monté en surface selon la revendication 1 dans lequel la couche de réservoir poreuse contient un agent actif.

14. Dispositif de distribution pouvant être monté en surface selon la revendication 1, dans lequel la couche de support contient un agent antimicrobien.

15. Dispositif de distribution pouvant être monté en surface selon la revendication 1, dans lequel la couche de réservoir comprend une composition liquide qui est incapable de s'écouler depuis la matière de réservoir poreuse jusqu'à ce que le réservoir soit comprimé.

16. Procédé consistant à empêcher la transmission de micro-organismes qui comprend l'utilisation ou l'application d'un dispositif de distribution pouvant être monté en surface selon n'importe laquelle des revendications 1 à 15.
